# EUROPEAN PATENT SPECIFICATION

(11) **EP 0 312 346 B1**
(45) Date of publication and mention of the grant of the patent: **17.08.1994**
(21) Application number: 88309582.0
(22) Date of filing: 13.10.1988
(51) Int. Cl.: C12N 9/52, C12P 21/06, C12N 15/00, C12N 9/99

(54) **E. coli sequence specific acid protease**
Sequenzspezifische saure E.coli-Protease
Protéase acide d'E. coli spécifique pour une séquence

(30) Priority: 16.10.1987 US 109796; 27.06.1988 US 211607
(43) Date of publication of application: 19.04.1989
(73) Proprietor: GENENCOR INTERNATIONAL, INC., South San Francisco, CA 94080 (US)
(72) Inventor: Ciccarelli, Richard Benjamin, Rochester New York 14650 (US); Holzschu, Donald Lee, Rochester New York 14650 (US)
(74) Representative: Brandes, Jürgen, Dr.

(56) References cited:
- EP-A- 0 089 626
- EP-A- 0 199 574
- EP-A- 0 212 914
- THE JOURNAL OF BIOLOGICAL CHEMISTRY, vol. 257, no. 18, 25. September 1982, pp. 11186-90 S. GOLTZ et al.
- BIOCHEMISTRY, vol. 26, no. 2, 27. January 1987, pp. 351-360, American Chemical Society, L.A. FISHEL et al.

## Description

### FIELD OF THE INVENTION

This invention relates to a novel E. coli protease, a method of cleaving peptides or proteins and a method for removing a peptide tag linked to a desired molecule.

### BACKGROUND OF THE INVENTION

Proteases are known for use in promoting general cleavage of proteins. Proteases are used in the food processing and cleaning industries. They can be used as catalysts in the drug and chemical industries, in generating polypeptides from cloned precursors, in destruction of waste products and in dissolution of blood clots. They are formulated with detergents or other surfactants in accord with methods known for use in industrial processes, especially laundry processes. In the laundry industry the proteases are combined with detergents, builders, bleach and/or fluorescent whitening agents. The proteases are used in the detergents to remove protein stains from fabrics and other surfaces.

Proteases are also used in other protein purification processes such as that disclosed in European Patent Application 0 150 126 discussed more fully hereinafter.

As more proteases are isolated, additional uses for them will undoubtedly be found.

E.coli is known to express several different proteins having some proteolytic activity. In general, E.coli proteases have not been well characterized and are not sequence specific. They degrade the entire protein into its constituent amino acids. An example of such proteases is disclosed in U.S. Patent 4,390,629. Also according to the literature, most of the E.coli proteases characterized to date are alkaline proteases.

The objective of the present invention is to provide proteases which are more sequence specific than prior art E.coli proteases, as well as methods that inhibit or prevent protein degradation by proteases.

### SUMMARY OF THE INVENTION

### 1. Protease

The present invention provides an acid protease of E.coli which catalyzes cleavage in proteins specifically between a) adjacent lysine sequences, b) adjacent arginine sequences, c) lysine-arginine sequences and d) arginine-lysine sequences. The production of this E.coli protease is induced in transformed E.coli according to the following method
a) providing a plasmid comprising the following operably linked genetic components;
   i) a cytochrome c peroxidase gene (CCP);
   ii) an E.coli promoter;
   iii) an E.coli origin of replication; and
   iv) an E.coli drug resistance gene;
b) transforming E.coli with a); and
c) culturing E.coli to over-express cytochrome c peroxidase.

It is believed that the new protease is produced by E.coli as a result of metabolic stress placed on the E.coli system resulting from over-expression of the protein cytochrome c peroxidase.

### 2. Brief Description of the Figures

Figure 1 is a schematic representation of the various steps included in the construction of the novel protein expression vector, pKH101, used to over-express CCP and induce E.coli to produce the protease of this invention.

Figure 2a-2g is a stepwise schematic illustration of the various construction operations carried out in adjusting the CCP gene relative to the E.coli promoter in intermediate plasmids to arrive at pKH101.

Figures 3-5 show a portion of the promoter and DNA sequences of pKH96, pKH98 and pKH101.

### DETAILS OF THE INVENTION

### 1. Materials

a) Reagents
   All reagents were purchased from standard commercial sources. Rabbit antisera against CCP (R-antiCCP) was obtained from Dr. J. Daiss of our laboratory. Oligonucleotides were synthesized by Mr. T. Cardillo of the University of Rochester, Dr. K. Jayaraman or Dr. S. Weber of Eastman Kodak Company.
b) Bacterial strains, plasmids and phage
   E.coli K12, strain JM103, obtained from T. Cardillo of the University of Rochester, (Δlac-pro, supE, thi, strA, endA, sbcB15/F'traD36, proAB, lacl^{Q}ZΔM15, P1 lysogen) was used to propogate plasmids, as a transformation recipient, and for screening of protein expression. E.coli strain TB1 (ara, Δlac, proAB, rpsL, hsdR, φ80 lacZΔM15) was obtained from Dr. S. Sliger of the University of Illinois and was used for protein production. Bacteriophage IR1, a variant of phage f1, was obtained from T. Cardillo of the University of Rochester. pEMBLYi32 was the gift of Prof. G. Cesareni of the European Molecular Biology Lab. YEp13CCP was obtained from Dr. J. Kaput of the University of Illinois. All strains were grown at 37°C in LB medium supplemented with ampicillin (100µg/ml).

### 2. Protein Expression Vector (pKH101)

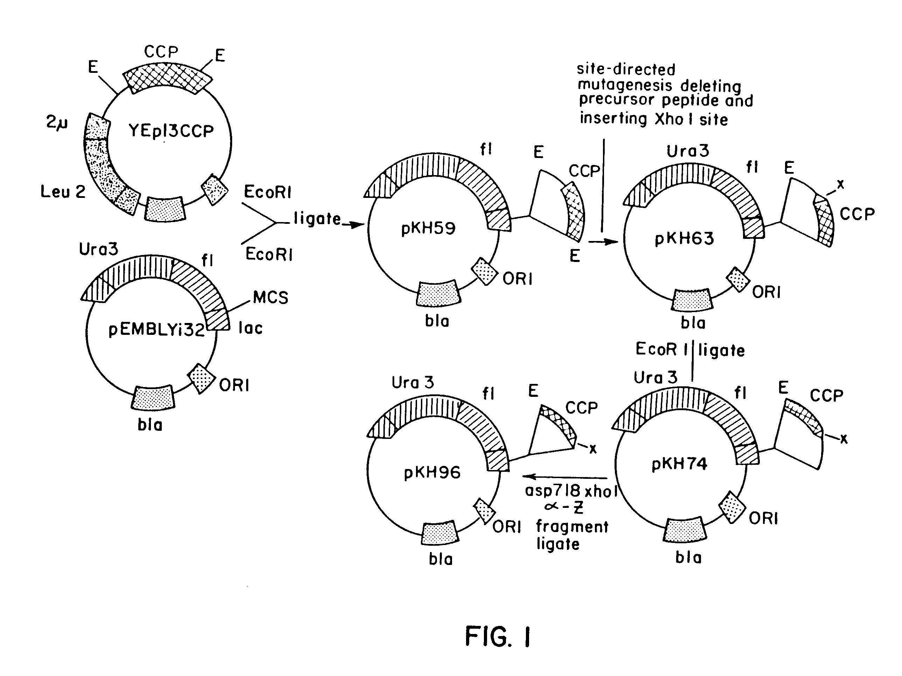

The protein expression vector of this invention is an E.coli plasmid, which 1) was used to induce the production of the protease of this invention in E.coli and 2) cause E.coli to over-express the protein cytochrome c peroxidase (CCP), has the following operably linked genetic components. The acronyms or names in parenthesis following each component are the symbols used for each component in figure 1.
A. A gene for cytochrome c peroxidase (CCP).
B. A lac gene sequence comprising a promoter and operator (LAC). Any of the E.coli promoters and operators known in the art will also be useful. See Nucleic Acids Research, vol. 15, no. 5 (1987) for an extensive list of other E.coli promoter DNA sequences. The lac gene used in pKH101 had the DNA sequences shown in figure 2g. Lac (P) promoter and operator (O) are regulatory DNA sequences which contain all the information that E.coli needs to recognize the CCP gene and express CCP. The promoter includes transcription control signals which instruct E.coli to start production of the protein. The CCP DNA sequences and the promoter DNA sequences are ultimately lined up in a way relative to each other so that when CCP mRNA is transcribed, E.coli ribosomes translate individual CCP codons in proper sequence. A codon is a set of three consecutive nucleotides which codes for a particular amino acid.
C. An E.coli origin of replication (ORI). This enables the plasmid to replicate in E.coli.
D. A β-lactamase gene (bla). Bla is a gene that codes for β-lactamase which confers ampicillin resistance to E.coli. Any E.coli drug resistant gene known in the art will also be useful. Drug resistance provides a means of selecting for the growth of cells containing plasmids having the drug resistance.
E. A phage origin of replication (f1). A phage is a virus that infects bacteria. During the replication cycle of an f1-type phage within E.coli, its DNA becomes single stranded. The phage origin of replication is used to make plasmid DNA single stranded when such plasmid is being replicated in E.coli. This is important for site-directed mutagenesis which is a process for specifically changing a nucleotide or a series of nucleotides.

### 3. General Method of Making pKH101

The protein expression vector, pKH101 was made using a combination of site-directed mutagenesis and standard recombinant DNA methods. pKH101, and variations thereof, was constructed from two extant plasmids: YEp13CCP from yeast containing the CCP gene and pEMBLYi32 containing the other enumerated genetic components.

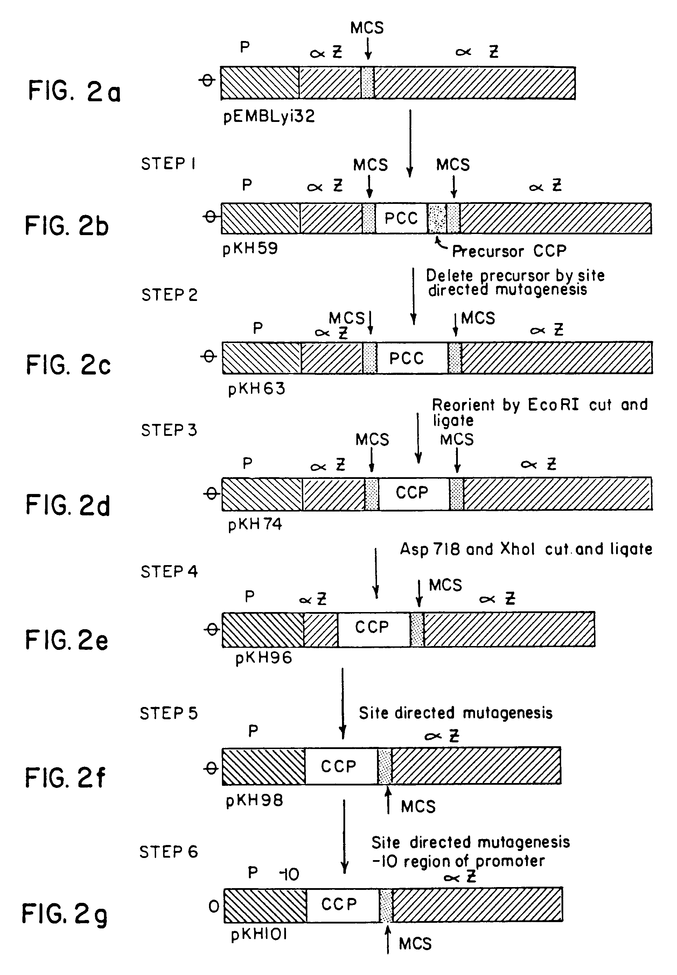

Figures 1 and 2 are a schematic illustration of the various steps carried out in the method to make pKH101. In general, the method involved:
a) removing the required genetic component from the starting plasmids (pEMBLYi32 and YEp13CCP);
b) combining the components in a single intermediate plasmid (pKH59);
c) conducting site-directed mutagenesis on pKH59 to remove a precursor portion of the yeast CCP gene, resulting in intermediate plasmid pKH63;
d) reorienting the CCP gene (pKH63) so that its DNA sequence is correct for expressing CCP, resulting in intermediate plasmid pKH74;
e) deleting a portion of the DNA sequence, by restriction enzyme digestion and subsequent ligation, between the promoter and the CCP gene as an intermediate step of putting the start of translation codon in proper sequence with the CCP gene, resulting in intermediate plasmid pKH96;
f) removing DNA sequences still remaining between the promoter and CCP genes in pKH96 by site-directed mutagenesis resulting in intermediate plasmid pKH98; and
g) changing DNA sequences in the promoter of pKH98 by site-directed mutagenesis, to enhance expression of CCP by E.coli, resulting in pKH101.

Protein expression vector pKH101 was sent to American Type Culture Collection on October 13, 1987. It received a deposit date of October 14, 1987, and a file designation of 67541.

### 4. Detailed Method of Making pKH101

### a) Construction of pKH59 (figure 1 and figure 2a, Step 1)

YEp13CCP was subjected to digestion with the restriction enzyme EcoRl to remove the CCP gene. This gene in yeast codes for a pre-apoprotein of 362 amino acids, including a 68 amino acid precursor. The entire gene was removed from YEp13CCP by a) growing the plasmid in E.coli, b) lysing of E.coli, c) isolating the plasmids and d) digestion of the plasmid with EcoRl. EcoRl cleaved the plasmid at a specific nucleotide sequence (GAATTC) indicated by "E" in YEp13CCP of figure 1 to give large and small DNA fragments. The small fragment contained the CCP gene.

Referring to figure 2a, at this point in the construction process the lac gene of pEMBLYi32 comprised an operator (O), a promoter (P), the alpha fragment from lac Z (α-Z). Within the α-Z fragment is a multiple cloning site (MCS). pEMBLYi32 was restriction digested with EcoRl. This cut the plasmid, making an opening within the multiple cloning site (MCS). MCS is a site which can be cut by a variety of different restriction enzymes, including EcoRl. Two pieces of DNA that have been cut with the same restriction enzyme can be combined using the enzyme, DNA ligase. Hence, ligating the CCP gene (which was cut out of YEp13CCP with EcoRl) with pEMBLYi32 (also cut with EcoRl) effectively inserted the CCP gene into MCS within the α-Z fragment (figure 2b). The foregoing digestion and ligation resulted in pKH59 (figures 1 and 2b).

### b) Construction of pKH63 from pKH59 (Step 2)

pKH59 was essentially pEMBLYi32 with the CCP gene from YEp13CCP in the pEMBLYi32 multiple cloning site. In this step the precursor DNA sequence on the CCP gene in pKH59 was deleted by site-directed mutagenesis to make pKH63 (figures 1 and 2c). pKH59 was first made single stranded. E.coli (JM103) was transformed with pKH59. JM103 was subsequently infected with a helper phage IR1 making pKH59 a single stranded phagemid particle. After E.coli growth was continued overnight, the cells were removed by centrifugation. The extracellular phagemid particles containing ssDNA (single stranded pKH59) were precipitated and purified, and stored according to Messing, "New MP Vectors for Cloning", pp. 20-78, Method in Enzymology, vol. 101, Academic Press, New York.

A synthetic piece of ssDNA (primer) was annealed to the single stranded pKH59 in the regions adjacent to the precursor CCP sequences designated for deletion. The primer was a) 50 bases long, b) complimentary to the DNA sequences adjacent to the deletion site and c) non-complimentary to the precursor DNA sequences of the CCP gene to be deleted. This resulted in a non-annealed loop comprising the precursor sequences (figure 2b, precursor CCP) to be deleted. Primer extension reactions using DNA polymerase large fragment were done by the method of Zoller using a single primer. Zoller et al, Oligonucleotide-Directed Mutagenesis, DNA3: pp. 479-88. Site-directed mutagenesis was used to delete the 201 bp between the initiation codon ATG and the amino terminus ATG of the mature protein and 2) insert an Xhol enzyme restriction site 8 nucleotides upstream of the start of protein translation.

Plasmids from eight colonies were isolated by alkaline extraction according to the techniques of Maniatus et al, Molecular Cloning, A Laboratory Manual, Cold Spring Harbor Labs, Cold Springs, New York. Each colony yielded two plasmids: One containing the original yeast CCP gene (pKH59, figure 2b) and a second plasmid containing the properly deleted precursor CCP gene (pKH63, figure 2c). Proper deletion of the precursor peptide gene segment was indicated by EcoR1-Xho1 fragments of 2.1 kb and 600 bp. pKH59 does not contain an Xho1 site as shown by restriction analysis. pKH63 contains Xho1 site ,thereby allowing it to be distinguished from pKH59. The properly deleted plasmids (pKH63, figure 2c) were purified by transformation of E.coli JM103 and identified by the presence of the Xho1 site. DNA sequencing confirmed that the proper deletion was made.

### c) Construction of pKH74 from pKH63 (Step 3)

pKH63 (figure 1 and 2c) had all the components of pKH59 except the precursor peptide of the original yeast CCP gene. However when pKH59 was made, the CCP gene was inserted in the multiple cloning site in the wrong orientation. This was determined by DNA sequencing. This improper orientation is shown as "PCC" in figures 2b and 2c.

Thus it was necessary to cut pKH63 in MCS with EcoRl; reorient and ligate the CCP gene back into lacZ. This was carried out as in Step 1. DNA sequencing confirmed that the right orientation of the CCP gene was made. This resulted in the plasmid pKH74 (figures 1 and 2d).

### d) Construction of pKH96 from pKH74 (Step 4)

pKH74 is exactly the same as pKH63 except the CCP gene has the right orientation for proper expression from the promoter by E.coli. Next it was necessary to fuse the promoter with the CCP gene. This was accomplished by deleting most of the unnecessary DNA sequences between the promoter and the start of translation of the CCP gene by restriction enzyme cleavage of pKH74 with Asp718 and Xho1 (figures 1 and 2e).

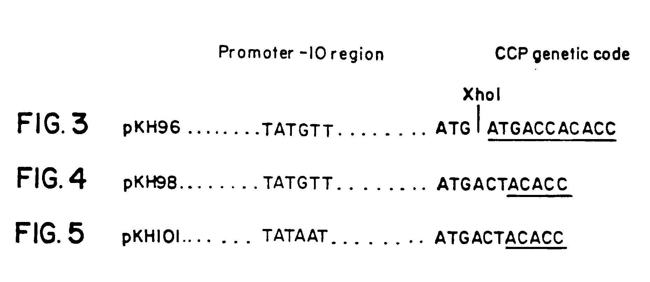

The cut ends of the resulting fragments were filled in with DNA polymerase. The remaining α-Z DNA sequences and the CCP gene were fused together with DNA ligase. This resulted in pKH96 (figures 1, 2e and 3). The fused DNA sequence of the promoter and the CCP gene on pKH96 are shown in figures 2e and 3. Note that pKH96 included an Xho1 site.

### e) Construction of pKH98 from pKH96 (Step 5)

In pKH96, the ATG of the CCP gene and the ATG of the promoter were separated by 63 bases. These remaining unwanted bases were deleted by site-directed mutagenesis on pKH96, using A 50 base oligonucleotide that exactly deleted from the sixth nucleotide of α-Z to the sixth nucleotide of CCP resulting pKH98 (figures 2f and 4). Following mutagenesis, plasmids from 8 colonies were analyzed for the presence of the Xho1 site, which should have been deleted in proper fusion of the promoter and CCP gene. One of these was taken as intermediate plasmid pKH98.

Figure 4, shows the relevant DNA sequences of pKH98. The DNA sequence of the CCP gene is underlined in figures 3, 4 and 5. pKH98 contained the start of translation codon ATG, in sequence with the first amino acid codon of the mature CCP gene, ACT. ACT is the codon for threonine. In pKH96 (figure 3), the CCP gene sequence started with ATGACC... In pKH98 (figure 4), ACT is substituted for ACC. Both are codons for threonine. pKH98 (figure 4) had the construction wherein promoter ATGACT replaced the ATGACC of the CCP gene. This plasmid, when transformed into E.coli strain JM103 provided CCP of proper size and sequence.

### f) Construction of pKH101 from pKH98 (Step 6)

To increase protein expression, the promoter region in pKH98 was changed using site-directed mutagenesis. By changing the promoter (from TATGTT to TATAAT) regions to E.coli consensus sequences at -10 bases, increased expression of CCP was achieved. Consensus sequences are those sequences generally recognized as forming strong promoter regions in E.coli. See figures 2g and 5. The change resulted in pKH101.

### 5. Expression of CCP in E.coli

Plasmid pKH101 was transformed into E.coli TB1 strain (obtained from Prof. Steve Sliger, University of Illinois). Expression of CCP of the correct size and sequence was confirmed by protein sequence analysis, ELISA, enzymatic activity and Western blots of SDS gels.

The TB1 strain containing pKH101 was grown and maintained on Luria Bertani (LB), ampicillin (amp) and 2% glucose plates. One loop of culture was then used to inoculate a starter culture of LB and amp. After 5 to 6 hours, this culture was used to inoculate a 5 liter fermenter (3X LB and amp and proline) or shake flask. The fermentation was run overnight at 37°C at a constant pH of 7.

The resulting E.coli cells were harvested and pelleted by centrifugation. The supernatant was discarded. CCP and the protease activity was obtained from the pellets according to the following purification procedures.

### 6. Purification of Expressed CCP

Two purification procedures were developed for isolation of CCP: procedure A at pH 5 and procedure B at pH 8. At pH 5, the novel acidic E.coli protease cleaved CCP between two lysines (lys 74 and lys 75) during the purification. At pH 8 the E.coli protease was inactive.

### a) Procedure A: pH 5

In this procedure, every step was carried out on ice. Each pellet was resuspended in 10-15 ml of lysis buffer having the following composition:
50 mM Sodium Acetate pH 5
0.1 mM sodium ethylenediaminetetracetate (Na₂ EDTA)
0.5 mM dithiothreitol (DTT)
0.2 mM phenylmethylsulfonyl fluoride (PMSF)
The pellets were resuspended and pooled.

The pooled cell pellets were lysed with an equal volume of glass beads (0.15µm) in a bead beater. The resulting lysate was treated with protamine sulfate (1-2 mg/ml) in order to precipitate nucleic acids. This did not remove any CCP from solution as determined by ELISA. Cellular debris and nucleic acids were then pelleted by centrifugation. The resulting soluble protein fraction (supernatant) contained apo-CCP, which was constituted to the mature protein with heme.

This soluble protein fraction was then left on ice for 30 minutes and then centrifuged at 30,000 x g for 20 minutes. The resulting cleared supernatant was then applied to a Q-Sepharose column (Pharmacia) equilibrated with 50 mM sodium acetate and 0.2 mM PMSF, pH 5.0. The loaded column was then washed with 50 mM sodium acetate pH 5. CCP was eluted from the column with a sodium acetate gradient at pH 5 as an impure mixture.

Fractions containing this mixture were combined, concentrated and desalted. The desalted solution was applied to a Biogel HT (Biorad) column equilibrated with 50 mM phosphate pH 6.

The column was washed with 100 mM phosphate, pH 6. CCP was eluted from the column with a linear phosphate gradient.

CCP was pooled and concentrated. Purity was confirmed by SDS electrophoresis, UV-Vis spectroscopy, isoelectric focusing and enzymology.

### b) Procedure B: pH 8

Procedure B was analogous to procedure A except as noted below.

After fermentation cells were resuspended in a pH 8 lysis (lysozyme) buffer having the following composition:
50 mM Tris-acetate pH 8.0
10 mM Na₂ EDTA
0.5 mM DTT
0.2 mM PMSF
lysozyme (hen egg white) 5 mg/ml
The resuspended cells were allowed to sit on ice 20-30 minutes (until viscous). They were then sonicated. Nucleic acids were precipitated. Protein lysate was prepared as in procedure A. The lysate was loaded onto a Q-Sepharose column as in procedure A, except that the column was pre-equilibrated with 50 mM Tris-acetate, pH 8.

The column was also washed with this same buffer. CCP was eluted with a linear NaCl gradient in tris-acetate at pH 8. The impure CCP mixture was treated as described in the pH 5 method.

### 7. Evidence of Protease Activity

Production of the novel acidic E.coli protease was induced during the expression of CCP by E.coli. The protease presented itself during purification of CCP.

It was observed that the "pH 5 method" routinely gave CCP of two forms - a native form of the expected 34 Kd MW; and a fragment of 26 Kd MW. When the purification was carried out at pH 8, only the 34 Kd native form was observed.

Also, Western blots revealed that both the 34 Kd form and 26 Kd form were recognized by an anti-CCP antibody. An E.coli protease, active at pH 5 but not at pH 8, was responsible for the generation of the 26 Kd fragment from the 34 Kd native protein.

Native radioactive carbon-14 labeled CCP was treated with E.coli (strain TB1) crude lysate. E.coli strain TB1 does not have the CCP gene unless it has been transformed with pKH101. The combined lysate (pH 5) and CCP were subjected to electrophoresis and fluorography. Only the 34 Kd CCP was observed (no proteolytic activity).

E.coli strain TB1 transformed with pKH101 was grown and crude lysate prepared (pH 5). The crude lysate was added to native radioactive labeled CCP. Both the 34 Kd forms and 26 Kd forms were observed after electrophoresis (proteolytic activity).

### PURIFICATION OF PROTEASE

The protease is associated with E.coli cell membranes. It is efficiently extracted from the membranes with the detergents sodium dodecyl sulfate (SDS) and lithium dodecyl sulfate (LDS). It is also partially extracted from the membranes with the detergents Triton X-100, Tween 80, and n-octyl glucoside.

The protease was purified by:
a) extraction of E.coli membranes with 0.5-1.0% LDS at 4°C in 50 mM hepes buffer, pH 7;
b) removal of membranous material by ultracentrifugation at 150,000 x g for 90 minutes at 4°C;
c) anion exchange chromatography (Q-sepharose) with the detergent Triton X-100 and a LiCl gradient (50-500 mM); and
d) gel exclusion chromatography (superose 12 FPLC) with the detergent SDS at 1%.

The fraction from step d) is a pure protease of approximately 50,000 MW and contains all of the sequence-specific protease activity described below.

The above purification procedure was carried out as follows.

One liter of E.coli, containing the CCP expression plasmid pKH101, was grown in LB/ampicillin media supplemented with proline at 37°C in a shake flask for 16 hours. Cells were harvested by centrifugation at 5000 x g. They were resuspended in lysis buffer (50 mM lithium acetate, 0.5 mM DTT, 1 mM EDTA, 0.2 mM PMSF, pH 5). The resuspended cells were lysed by sonication at 0°C. The cellular debris fraction containing the membrane-associated protease was pelleted from the lysate at 30,000 x g at 4°C. This pellet was resuspended in lysis buffer containing 0.5% (w/v) lauryl sulfate detergent (sodium salt, SDS; or lithium salt, LDS). This suspension was stirred for 60 minutes. A detergent-solubilized crude protease fraction was then prepared by pelleting the membranes by ultracentrifugation for 60 minutes at 150,000 x g. The nonionic detergent Triton X-100 was added to 0.5%. This fraction was dialyzed against 0.5% Triton X-100 in 50 mM hepes (pH 7) for 12 hours. The dialyzed solution was centrifuged at 30,000 x g. The centrifuged solution was applied to an anion exchange column (Q-sepharose, Pharmacia) at pH 7. The column was eluted with a linear salt gradient (50 mM hepes, 0.5% Triton X-100, 50-500 mM LiCl, pH 7). The partially pure protease eluted from this column at approximately 400 mM LiCl. The fractions containing the protease were pooled and dialyzed against 50 mM sodium acetate plus 0.05% Triton X-100, pH 5 (several changes over 48 hours). The protease was concentrated ten-fold with an Amicon ultrafiltration cell. The protease was purified to homogeneity by gel filtration chromatography on Superose 12 FPLC in the presence of 50 mM sodium acetate and 1.0% SDS. The resulting protein of approximately 50,000 MW (as measured by SDS gel electrophoresis) was dialyzed and stored at -20°C. The sequence specific protease activity was monitored throughout this procedure using CCP as a substrate with the following assay.

Protease Assay - The protease was assayed by combining in 1.5 mL microtubes 6 µL protease fraction, 3µL CCP (5 mg/ml), 3 µL 500 mM sodium acetate at pH 5, followed by 30 minutes incubation at 37°C. This mixture was electrophoresed on 12.5% acrylamide-SDS gels, and proteolytic activity was evidenced by the appearance of the specific 26 kd degradation product from native CCP.

### IDENTIFICATION OF PROTEASE ACTIVITY

### 1. Sequence Specific Cleaving Activity

To identify the proteolytic sequence specificity in CCP, the 26 Kd fragment was separated from the 34 Kd fragment using hydrophobic interaction chromatography (FPLC - phenyl superose column).

Both the 26 Kd fragment and the 34 Kd native protein were sent to the University of California, San Francisco, for N-terminal sequencing. The 26 Kd fragment had lysine at the N-terminus. Comparison with the native sequence indicated that the protease cleaved CCP between position 74 (lys) and position 75 (lys), i.e. between adjacent lysines. CCP has only one lys-lys sequence.

Using site-directed mutagenesis, which allowed for specific single changes in the amino acid sequence of a protein, mutations were made at the lys 74-lys 75 site of CCP. At pH 5, these mutations, which changed either of the lysines to threonine, or both lysines to threonine, gave new forms of CCP which were not cleaved by the protease at pH 5 as determined by gel electrophoresis and Western blots. The protease does not cleave when one or both of the lysines at its target recognition sequence are changed to threonine. Each mutant was confirmed by DNA sequence analysis.

Other mutations were made in the amino acid sequences at the lys 74-lys 75 site in CCP. The protease was found to specifically cleave between the following amino acid sequences in CCP:
arginine 74 - arginine 75;
arginine 74 - lysine 75; and
lysine 74 - arginine 75.

The protease is active (pH 4-6 at 4-37°C) in up to 0.5% of SDS, LDS and Triton X-100 (a trademark of Rohm and Haas of Philadelphia for octylphenylpoly[ethylene glycol ether]ₙ.

### 2. Inhibition of the protease cleaving activity

Experiments showed that the protease activity was not inhibited by EDTA, PMSF or DTT, but was inhibited by poly-L-lysine and poly-L-arginine.

E.coli expressing CCP was lysed at pH 5 in the presence of increasing concentrations of poly-L-lysine or poly-L-arginine. Any protease activity was evidenced by the appearance of the 26 Kd fragment on Western blot. 1 mM poly-L-lysine or poly-L-arginine completely inhibited the protease activity. No 26 Kd fragment appeared; DTT, PMSF and EDTA had no inhibitory effect on the lysine-lysine lysine-arginine, arginine-lysine or arginine-arginine cleaving activity of the protease.

### UTILITY OF PROTEASE

The protease of this invention is useful in an affinity purification process for proteins which is disclosed in European Patent Application 0 150 126 published July 31, 1985. In the process a ligand is employed to isolate and purify substantially all protein molecules expressed by transformed host cells. The process can be used on a small research level or a large commercial scale to purify substantially all protein molecules produced by recombinant DNA techniques.

In the disclosed process a hybrid molecule composed of protein and an identification or marker peptide is produced by recombinant DNA techniques. The identification peptide ideally includes two primary components: a highly antigenic N-terminal portion and a linking portion to connect the identification peptide to the protein.

The linking portion of the identification peptide is
cleavable at a specific amino acid residue adjacent to the protein molecule by use of a sequence specific proteolytic agent. The hybrid identification peptide/protein molecules expressed by the transformed host cells can be isolated by affinity chromatography techniques. This is accomplished by constructing an affinity column with immobilized ligands specific to the antigenic portion of the identification peptide thereby binding the expressed hybrid identification peptide/protein molecule.

The bound identification peptide/protein molecules can be liberated from the column and then the identification peptide cleaved from the protein molecule with an appropriate proteolytic agent such as the protease of this invention. The cleaving releases the desired protein molecule. The linking portion of the identification peptide is composed of amino acids that are recognized by the sequence specific proteolytic agent which cleaves the linking portion at a location adjacent the protein molecule. Ideally, the amino acid sequence of the linking portion is unique, thus minimizing the possibility that the proteolytic agent will cleave the protein molecule.

The protease of the present invention would be useful in this disclosed process in circumstances where the amino acid sequence between the linking portion of the identification peptide and the protein is a sequence recognized by the protease. The protein molecule may be composed of any proteinaceous substance that can be expressed in transformed host cells.

Proteins contemplated by EPO 0 150 126 include enzymes, whether oxidoreductases, transferases, hydrolases, lyses, isomerases or ligases;
storage proteins, such as ferritin or ovalbumin or transport proteins, such as hemoglobin, serum albumin or ceruloplasmin; proteins that function in contractile and motile systems, for instance, actin and myosin;
proteins that service a protective or defense function, such as the blood proteins thrombin and fibrinogen; binding proteins, such as antibodies or immunoglobulins that bind to and thus neutralize antigens;
toxic proteins, such as ricin from castor bean or grossypin from cotton linseed; and
proteins that serve as structural elements such as collagen, elastin, alpha-keratin, glyco-proteins, virus-proteins and muco-proteins.

In addition to the above-noted naturally occurring proteins, synthetic proteins defined generally as any sequence of amino acids not occurring in nature are also contemplated.

Genes coding for the various types of protein molecules identified above may be obtained from a variety of prokaryotic or eukaryotic sources, such as bacterial, plant or animal cells. The genes can be isolated from the chromosome material of these cells by employing standard, well-known techniques. A variety of naturally occurring and synthesized plasmids having genes coding for different protein molecules are now commercially available from a variety of sources. The desired DNA also can be produced from mRNA by using the enzyme reverse transcriptase. This enzyme permits the synthesis of DNA from an RNA template.

Proteases are also known for use in efforts in the pharmaceutical industry to manufacture hormones by using protease to cleave precursor peptides away from the active hormone.

## Claims

1. An acid protease of E. coli which catalyzes cleavage in proteins specifically between a) adjacent lysine sequences, b) adjacent arginine sequences, c) lysine-arginine sequences and d) arginine-lysine sequences, which can be prepared from E. coli by a method comprising the steps of
a) providing a protein expression vector comprising the following operably linxed genetic components;
i) a cytochrome c peroxidase gene;
ii) an E. coli promoter;
iii) an E. coli origin of replication; and
iv) an E. coli drug resistance gene;
b) transforming E. coli with a); and
c) culturing E. coli to express cytochrome c peroxidase.

2. The protease of claim 1 wherein the cleavage occurs at about pH 5.

3. The protease of claim 1 wherein the cleaving ability of the protease is inhibited by poly-L-lysine or poly-L-arginine.

4. The protease of claim 1 wherein the cleaving ability of the protease is uninhibited in the presence of phenylmethylsulfonyl fluoride (PMSF), ethylenediaminetetraacetic acid (EDTA), or dithiothreitol (DTT).

5. The protease of claim 1 having cleaving activity in up to 0.5% sodium dodecyl sulfate, lithium dodecyl sulfate or octylphenylpoly[ethylene glycol ether]ₙ.

6. The protease of claim 1 in the form of a pure protein of approximately 50,000 MW.

7. A method of cleaving peptides or proteins between a) adjacent lysine sequences, b) adjacent arginine sequences, c) lysine-arginine sequences and d) arginine-lysine sequences comprising the step of digesting the peptide or protein in an acid protease according to claim 1, 2, 3, 4, 5 or 6.

8. A method of removing a peptide tag linked to a desired molecule through a) adjacent lysine sequences, b) adjacent arginine sequences, c) lysine-arginine sequences and d) arginine-lysine sequences comprising the step of treating the molecule in an acid environment with an acid protease of E.coli according to any one of claims 1 to 6.

9. The method of claim 8 wherein the molecule is a protein or a hormone.

10. The protease of claim 1 wherein the protein expression vector includes an E.coli lac promoter and the drug resistant gene is β-lactamase.

## Patentansprüche

1. Säure-Protease von E. coli, welche die Spaltung in Proteinen speziell zwischen a) benachbarten Lysin-Sequenzen, b) benachbarten Arginin-Sequenzen, c) Lysin-Arginin-Sequenzen und d) Arginin-Lysin-Sequenzen katalysiert, und die aus E. coli nach einem Verfahren hergestellt werden kann, das die folgenden Stufen umfaßt:
a) Bereitstellung eines Protein-Expressions-Vektors mit den folgenden innerhalb eines Operons verknüpften genetischen Komponenten:
i) einem Cytochrom c Peroxidasegen;
ii) einem E. coli-Promoter;
iii) einem E. coli-Replikations-Origin; und
iv) einem E. coli eine Arzneimittel-Widerstandsfähigkeit bewirkenden Gen;
b) Transformierung von E. coli mit a); und
c) Kultivierung von E. coli zum Zwecke der Exprimierung von Cytochrom c Peroxidase.

2. Protease nach Anspruch 1, bei der die Spaltung bei einem pH-Wert von etwa 5 erfolgt.

3. Protease nach Anspruch 1, bei der die Spaltungsfähigkeit der Protease durch Poly-L-lysin oder Poly-L-arginin inhibiert wird.

4. Protease nach Anspruch 1, bei der die Spaltungsfähigkeit der Protease in Gegenwart von Phenylmethylsulfonylfluorid (PMSF), Ethylendiamintetraessigsäure (EDTA) oder Dithiothreitol (DTT) nicht inhibiert wird.

5. Protease nach Anspruch 1 mit einer Spaltungsaktivität in bis zu 0,5%igem Natriumdodecylsulfat, Lithiumdodecylsulfat oder Octylphenylpoly[ethylenglykolether]ₙ.

6. Protease nach Anspruch 1, in Form eines reinen Proteins eines Molekulargewichtes von ungefähr 50000.

7. Verfahren zur Spaltung von Peptiden oder Proteinen zwischen a) benachbarten Lysin-Sequenzen, b) benachbarten Arginin-Sequenzen, c) Lysin-Arginin-Sequenzen und d) Arginin-Lysin-Sequenzen mit der Stufe der Digestierung des Peptides oder Proteins in einer Säure-Protease gemäß Anspruch 1, 2, 3, 4, 5 oder 6.

8. Verfahren zur Entfernung eines Markerpeptides, das an ein gewünschtes Molekül gebunden ist durch a) benachbarte Lysin-Sequenzen, b) benachbarte Arginin-Sequenzen, c) Lysin-Arginin-Sequenzen und d) Arginin-Lysin-Sequenzen mit der Stufe der Behandlung des Moleküls in einer sauren Umgebung mit einer Säure-Protease von E. coli nach einem der Ansprüche 1 bis 6.

9. Verfahren nach Anspruch 8, bei dem das Molekül ein Protein oder ein Hormon ist.

10. Protease nach Anspruch 1, in der der Protein-Expressions-Vektor einen E. coli lac-Promoter einschließt, und in der das bezüglich Arzneimitteln resistente Gen β-Lactamase ist.

## Revendications

1. Protéase d'acide de E. coli qui catalyse le clivage de protéines spécifiquement entre a) des séquences adjacentes à la lysine, b) des séquences adjacentes à l'arginine, c) des séquences lysine-arginine et d) des séquences arginine-lysine, qui peut être préparée à partir de E. coli par un procédé comprenant les étapes suivantes :
a) apport d'un vecteur d'expression de protéine contenant les composants génétiques suivants fonctionnellement liés :
i) un gène de cytochrome c-peroxydase;
ii) un promoteur de E. coli;
iii) une origine de réplication de E. coli;
iv) un gène de résistance à un médicament de E. coli;
b) transformation de E. coli avec a) ; et
c) culture de E. coli pour exprimer la cytochrome c peroxydase.

2. Protéase selon la revendication 1, dans laquelle le clivage apparaît à un pH d'environ 5.

3. Protéase selon la revendication 1, dans laquelle la capacité de clivage de la protéase est inhibée par une poly-L-lysine ou une poly-L-arginine.

4. Protéase selon la revendication 1, dans laquelle la capacité de clivage de la protéase n'est pas inhibée en présence de fluorvre de phénylméthylsulfonyle (PMSF), d'acide éthylènediaminetétraacétique (EDTA) ou de dithiolthréitol (DTT).

5. Protéase selon la revendication 1, présentant une activité de clivage dans jusqu'à 0,5 % de dodécylsulfate de sodium, de dodécylsulfate de lithium ou d'octylphénylpoly[éther d'éthylèneglycol]n.

6. Protéase selon la revendication 1, sous forme d'une protéine pure de masse moléculaire d'environ 50 000.

7. Procédé de clivage de peptides ou de protéines entre a) des séquences adjacentes à la lysine, b) des séquences adjacentes à l'arginine, c) des séquences lysine-arginine et d) des séquences arginine-lysine contenant l'étape de digestion du peptide ou de la protéine par une protéase acide selon la revendication 1,2,3,4,5, ou 6.

8. Procédé d'élimination d'un marqueur peptidique lié à une molécule souhaitée dans a) des séquences adjacentes à la lysine, b) des séquences adjacentes à l'arginine, c) des séquences lysine-arginine et d) des séquences arginine-lysine contenant l'étape de traitement de la molécule dans un environnement acide par une protéase acide de E. coli selon l'une quelconque des revendications 1 à 6.

9. Procédé selon la revendication 8, dans lequel la molécule est une protéine ou une hormone.

10. Protéase selon la revendication 1, dans laquelle le vecteur d'expression de protéine contient un promoteur lac de E. coli et en ce que le gène de résistance à un médicament est la pénicillinase.
